# EUROPEAN PATENT SPECIFICATION

(11) **EP 1 424 938 B1**
(45) Date of publication and mention of the grant of the patent: **13.08.2008**
(21) Application number: 02707391.5
(22) Date of filing: 14.03.2002
(51) Int. Cl.: A61B 5/11

(54) **METHOD AND DEVICE FOR SELECTIVE AND NON-INVASIVE DETECTION OF SKELETAL MUSCLES CONTRACTION PROCESS**
VERFAHREN UND VORRICHTUNG ZUR SELEKTIVEN UND NICHTINVASIVEN MESSUNG DES ABLAUFS DER SKELETTMUSKELKONTRAKTION
METHODE ET APAREILLE DE DETECTION SELECTIVE ET NON-INVASIVE DU PROCESSUS DE CONTRACTION DES MUSCLES DU SQUELETTE

(30) Priority: 21.03.2001 SI 200100081
(43) Date of publication of application: 09.06.2004
(73) Proprietor: TMG-BMC D.O.O., 1000 Ljubljana (SI)
(72) Inventor: VALENCIC, Vojko, 1000 Ljubljana (SI)
(86) International application number: PCT/SI2002/000007
(87) International publication number: WO 2002/074167

(56) References cited:
- FR-A- 2 612 068
- GB-A- 2 302 949
- US-A- 4 688 581
- US-A- 6 063 044

## Description

### Technical field

Biology, biomechanics, measuring technique.

### Description of the problem

Daily work in fields of physiotherapy, professional sport, etc. usually requires muscle force estimation or torque about specific joint and distinguishing a single-muscle contraction from a muscle-group contraction (selectivity), as well. Skeletal muscles are considered an integrated system of a human body and interfering with it in an invasive way is often in contradiction with the aim of the measuring data collecting procedure (e.g. it is senseless to carry out biopsy on sportsmen - the process of taking off a muscle-fibre-sample would only cause them a lot of pain and demand a minimum sport activity in order to let the wound heal properly).

The following device, described in this document, enables completely non-invasive and selective measurements of dynamic and contractile properties of skeletal muscles.

### State of the art

Estimation of muscle-fibre-type percentage is usually assessed by applying histochemical and immunocytochemical techniques. Both techniques are based on myofibrillar adenosinetriphosphatase (M-ATP-aze) activity, which is founded on works by R. Close (1965) and M. Baranyja (1967). Both techniques are applied on samples obtained by muscle biopsy and are therefore considered invasive and not suitable for routine usage. With respect to these conclusions Burke et al. (1976) suggested motor unit classification in cat skeletal muscles according to physiologically defined contraction time in non-tetanic contraction fatigue test. Test results indicated following classification: type I (slow-twitch, fatigue resistant), type IIa (fast-twitch, fatigue-resistant) and type IIb (fast-twitch, fast fatiguable) muscle fibres. The distinction between type I and type II muscle fibres is based on metabolic properties, excitability, resistance to fatigue and twitch response to single electrical stimulus. The study on isolated rat-, mice- and hamster muscles (Wetzel and Gros 1998) revealed that according to predominating muscle fibre types there is a correlation between muscle force and intracellular calcium concentration. Muscle fibre types differentiation starts on the level of embryo (in the first few weeks) when the fibres are in the process of growing and innervating; this indicates the predetermination of our skeletal muscles composition. It is, however, possible for muscle fibres to change due to neuromuscular diseases, sports training, denervation or electrical stimulation.

Until now accurate and direct measurements of skeletal muscles functioning and their properties have been performed in an invasive way. Direct measurements of biomechanical- (e.g. muscle force (Buschman et.al. 1996)), mechanical- (Rassier, 1998) and contractile properties (Olson & Marsh, 1992) carried out so far have been performed only on isolated animal muscles. Biomechanical properties in human skeletal muscles have usually been detected indirectly by measuring muscle force or torque about a specific joint. Clarkson and Gilewich (1989) have defined "muscle strength" as maximum value of torque (exerted by a group of muscles) or muscle force (exerted by a single muscle) in maximum voluntary contraction (MVC) under specific conditions (type of contraction, joint angle). To perform such (direct) measurements properly it would be necessary to attach the measuring mechanism to a muscle tendon. Although such measurements were accomplished (Komi, 1990) they haven't been applicable neither in clinical- nor in sport-practice. Therefore muscle force exerted by a single muscle or a group of muscles is still assessed by using the above described indirect methods (mostly with mechanical braces).

Variety of muscle fibres increases the range of demands that the muscle can accommodate. This accommodation is enabled by a joint functioning of several muscles which are divided in two groups depending on the way they perform the movement about a specific joint: 1) extensor(s) is a muscle (or a group of muscles) which produces extensile joint movement; 2) flexor(s) is a muscle (or a group of muscles) which is active in an opposite direction (joint flexion). Synergysts are subsidiary muscles helping flexors to achieve a desired muscle torque, improve the movement coordination and stabilize the joints.

It has been mentioned above how very important are the measuring conditions under which the measurements of biomechanical properties of skeletal muscles are carried out. The factors that have impact on measuring results are as follows:
1) type of movement: dynamic, isometric, static, isotonic, concentric, eccentric, isokinetic, and isoinert - all defined in Kroemer (1991);
2) nervous system: the ability to synchronously activate larger number of motor units, which is also dependent on the subjects' work out status;
3) mechanical brace: lay, type of attachment and rigidness of mechanical brace impact the repeatability, variability and thus standardisation of the measuring method.
4) position of the measuring subject (relation between the muscle tension and length (Soderberg, 1992)).
   In case the muscle is located in a multiple joint system, the problem of providing proper measuring conditions is even more complex.

From the preliminary studies (Sukop & Nelson, 1974) it has been concluded that muscle force is proportional to contraction time (time period which starts at the beginning of contraction and ends when maximum muscle force is achieved) - the longer the contraction time the greater the muscle force (force-time relationship).
Measurements performed with mechanical braces are also affected by: motivation, sex, age, tiredness, time of the day when the measurements are performed, temperature in the measuring room, profession and muscle contractile properties which also vary according to muscle injury, immobilization, inactivity, paralysis and other pathologic changes in neuro-muscular system.
In clinical environment manual and functional muscular test (MMT, FMT, Admudsen, 1990) are most frequently used. Yet (non-invasive) measuring muscle force and muscle torque provides the most accurate biomechanical properties estimation of skeletal muscles.

Measuring methods for muscle force or muscle torque determination still represent a technical problem, which - to some extent - has been solved and results in various mechanical braces and procedures (for estimation of skeletal muscles mechanical properties) which have been divided in two groups: 1) first one includes local muscular groups measurements (e.g. elbow flexors) and 2) the other one includes several muscle groups measurements, all of them involved in more complex movements such as getting up. Despite all, none of the measuring methods features a dominant advantage that would make it generally applicable.
In clinical environment manual muscular test (MMT) is preferred for its simplicity and short measuring procedure. It is carried out under isometric conditions by limb fixation. Measuring results are subjective and of poor selectivity, but can to some extent be improved either by methods for data processing (modelling, etc.) or by completion of the measuring equipment with measuring instruments and devices for measuring muscle strength (Amundsen, 1990). One of the methods used is lifting a weight of maximum weight (1-time or 10-times) under standardized conditions. Measuring results inform us only about activated muscles (no data on contraction velocity or selectivity what so ever).

Manual dynamometer: the measuring device is based on hydraulic, spring or strain gauge principle and is held by the measurer. Subject performs a pull at MVC against the measurer. Measurement is carried out under isometric or eccentric conditions. The device is portable, relatively inexpensive and within commercial reach. Its main disadvantage is non-selectivity and limb fixation, which makes the measuring process subjective (measurer dependent). Moreover, no data on muscles dynamic properties can be obtained (very important with persons with diminished motor capabilities).

Wire-tensiometer: with this device isometric muscle force is measured by one end of the wire being attached to fixed holder and the other one to the limb of the subject. It is mainly used for research purposes (research laboratories), it is inexpensive and provides reliable measuring results in healthy subjects. Its disadvantage is non-selectivity and not enough high sensitivity when muscle force is very low. Again no data on muscles dynamic properties can be obtained.

Mechanical braces (strain gauge principle): strain gauges are attached to metal ring or stick. Isometric muscle force is measured by strain gauge mechanical deformation, which causes alteration of electrical conductivity. Main disadvantage of this device is its frequently required calibration and sensitivity to temperature changes. Method accuracy is often lowered due to difficulties with placing the brace on the measured limb. This method is therefore rather seldom used in clinical environment and also rarely within commercial reach due to device's high price.

Isokinetic dynamometer: is used for (greater) muscle force measurements at joint rotation with constant speed (0 ° in 500° per second). Movements are performed either under isokinetic-concentric or isokinetic-eccentric conditions. In clinical environment this method is used for therapeutic as well as exercise purposes. The device itself is quite big and expensive. Calibration with external weights is required. In order to perform measurements correctly, the device axes have to fit joint axes, which is almost impossible to achieve. This method is a non-selective one and cannot be applied if the subject is very tall or short.

Another large group of devices for measuring biomechanical properties of skeletal muscles is used for detecting body-movement velocity or movements of specific parts of the body. The velocity parameter is present in our everyday activity, controlled and regulated in sport activity and might as well change in subjects with neuromuscular system malfunction.

Movement velocity is determined by muscle contraction velocity, which is connected with the speed of generating inner muscle tension and all together depends on muscle fibres composition. Many other factors have impact on movement velocity: mass of body segments, muscle length, physical condition, body and outer temperature, inner frictions, gravity and other physiological factors that have already been presented.

Velocity parameter is obtained by measuring velocity of linear movements (of specific body segments) or by measuring torque about specific joint against time. Some devices and instruments for velocity measurement: force transducers, electrogoniometric systems, opto-electronic and video devices for movement monitoring and recording, cinematographic techniques and accelerometric devices, where velocity is calculated by integrating measured acceleration. Accelerometers for devices with three-dimensional detection system are distinguished for very easy attachment on human body parts. Price of such devices is quite high but still acceptable.

In order to determine force-velocity relation it would be necessary to define a burden used. Unfortunately there are many factors - anatomical, anthropological and technical (synchronisation between instruments, resolution, vibration, etc.) - that prevent us from defining it. This further on leads to inaccuracy in obtaining measuring results and is described as a very specific technical problem (Robertson & Springs, 1987).

Another biomechanical parameter often observed, is fatigue parameter which is closely related to muscle endurance. There are three types of muscle endurance: (1) physiological-, cardiovascular- and neuromuscular-dependent endurance - known as general endurance, (2) absolute endurance, defined as a period of time in which neuromuscular system exerts muscle force big enough to resist a constant burden irrespective of muscle strength and (3) relative endurance - defined the same as absolute endurance and normalized with muscle strength. There is a correlation among the three endurance types and contraction type: (a) there is a statistically significant correlation between isotonic muscle strength and absolute isotonic endurance and (b) there is a statistically significant correlation between muscle strength and relative muscle endurance. Similar are the relations for isometric conditions (Jensen & Fisher, 1979).

Muscle endurance is mainly dependent on muscle fibres composition. Three different types of muscle fibres are comprised in each skeletal muscle: type I (slow-twitch, fatigue resistant, oxidative), type IIa (fast-twitch, fatigue-resistant, oxidative-glycolytic) and type IIb (fast-twitch, fast fatigable, glycolytic) muscle fibres. For estimation of local muscle endurance it is recommended for muscle contraction to last more than 10 seconds and less than 2 minutes (Åstrand & Rodal, 1977) otherwise type I and type IIa fibres would be affected too. 2 - 6 minute submaximum dynamic voluntary contractions would cause type IIa and type I fibres to fatigue as well. When fatigue process is monitored, the following factors should be taken into account: blood circulation, burden, neuromuscular mechanisms, contraction force and test repetition frequency.

Endurance informs us how long certain muscle activity can be retained - therefore it is expressed as a time value. Retaining certain muscle force (muscle activity) requires standardization, measurement and control over other factors: muscle force or muscle torque, joint movement velocity and joint angles. The level of endurance can also be expressed as number of contraction repetitions before maximum torque or maximum force value diminishes by 20%, 25% or 50%. However, this definition of endurance prevents us from distinguishing absolute from relative endurance (as defined above): it is often the case that an elderly subject or subject with specific muscle atrophy performs more repetitions (before 50% decrease) than a young, healthy subject. Endurance is sometimes measured even through work done at isokinetic repetitions (usual number of repetitions 25), but cannot be applied in isometric contractions since there is no direct muscle work detected.

Muscle activity in terms of cellular activity is often limited by specially prepared animal skeletal muscles (Jackman, 1997). They are sometimes used as a comparison to human skeletal muscles after applying non-invasive measuring methods (Mietysch et al, 1998). Other biomechanical properties, such as muscle force (Buschman et al, 1996), mechanical properties (Ettema, 1996, James et al, 1994), contractile properties (Olson & Marsh, 1993), etc. are in most cases studied on isolated animal muscles. Biomechanical and dynamic properties of skeletal muscles in humans are measured either indirectly and non-invasively (e.g. muscle force, muscle torque) or directly but invasively - invasive measuring methods are mostly applied on cadavers (Chan, 1998, Mitsiopoulos, 1997) if all the ethical requirements are met. Estimating muscle structure (with respect to muscle fibres composition) with histochemical analysis in human skeletal muscles is nowadays limited and focused on pathologically affected muscles (Castro, 1998).

Muscle activity is sometimes non-invasively estimated by monitoring specific muscle during the contraction process using computer tomography (CT) or magnetic resonance imaging (MRI) (Mietysch et al, 1998, Mitsiopoulos, 1997).

Scientists and medical doctors are often interested in muscle functioning. They observe force development during process of contraction by either indirect (mainly muscle torque (Donaldson et al, 1999)) or direct measurements - in most cases electrical activity is monitored by electromyiogram (EMG) (Siegler, 1982).

Torque measurement on one hand, is non-selective, requires various measuring braces in order to fit each joint, but still provides information on muscle force (Donaldson et al, 1999). Electromyographic measurement (EMG) on the other hand, is selective but instead of information on muscle force, it only informs us about motor unit recruitment. Although there is a correlation between EMG and muscle force, it varies dependent on the muscle, joint angle, etc. (Caldwell, 1989).

For indirectly collected data processing, mathematical models are often used. These models are based on biomechanical and dynamic properties of animal skeletal muscles and illustrate skeletal muscle functioning. For proper EMG signal processing specific mathematical analysis is required (Saitou et al. 1999). Patent Application GB-A-2 302 949 describes use of Hall effect displacement sensor separated from the skin of the subject by foamed polyurethane measuring muscle movement or arm rotation.

### Description of new solution

The patent presented includes measuring procedure and measuring device for selective and non-invasive measurements of skeletal muscles contractile and dynamic properties. For the purpose of this patent application this device was called tensiomyographic device and measuring procedure tensiomyography or shorter TMG. Measuring technique (measuring device and measuring procedure) was devised for prevention of invasive or indirect measurements of biomechanical, dynamic and contractile properties of human skeletal muscles; such measurements are presented in introduction and still represent a technical problem.

When muscle is contracted, its middle part - muscle belly - is thickened. Measuring technique is based on selective measurement of muscle belly displacement. From preliminary studies it has been concluded that muscle belly displacement is proportional to muscle force. Muscle belly displacement can be achieved either in voluntary or in electrically evoked muscle contraction. Measuring result (also called TMG response) informs us about biomechanical, dynamic and contractile properties of measured skeletal muscle or muscle group. Physical law states that the activity (work) that a muscle performs is proportional to production of muscle belly displacement and muscle force - therefore also proportional to the square of muscle belly displacement. Proposed measuring technique enables a comparison of specific muscles according to measured parameters e.g. contraction velocity, level of sustained contraction, relaxation velocity, muscle fibres composition, maximum muscle force and endurance parameter. Regarding state of the art in measuring techniques tensiomyographic technique has quite a few advantages over above mentioned techniques: apart from selectivity, which is considered its main advantage, tensiomyography features very simple calibration and usage. Tensiomyographic measurements can be carried out in combination with mechanical braces or other devices (within commercial reach), under isometric, isotonic or isokinetic conditions, while isometric conditions are preferred. Adjectives isometric, isotonic and isokinetic are used as defined in terms of biomechanics.

All measuring devices (invasive or non-selective) itemized so far, can easily be replaced by tensiomyographic device, the subject of invention.

Selectivity is the main advantage of tensiomyographic measuring technique. Parameters that can be measured indirectly (muscle torque, body segments acceleration and joint angles) are detected as a result of muscle group activation. Tensiomyographic measuring technique features high selectivity - biomechanical, contractile and dynamic properties can be monitored in either single muscle or group of muscles.

Next advantage over mechanical braces is that measurements in all surface skeletal muscles are performed with the same measuring equipment. In case of mechanical braces for each joint specially designed brace is required.

Apart from non-invasiveness, selectivity and simple appliance, tensiomyographic device also offers high sensitivity, which enables detection of weak contractions. Such contractions are produced by muscles, weakened due to neuromuscular diseases, denervation or muscle atrophy (inactivity). By torque measurement, for instance, such contractions cannot be detected nor monitored.

The main idea for tensiomyographic measuring method arose when biomechanical responses in children with muscle dystrophy were observed and insufficient measuring equipment was at hand (Valen 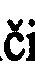 i 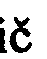 1990). In clinical practice muscle response to single electrical stimulus is used to estimate the muscle dystrophy progress. Initial phase of this disease harms tibialis anterior muscle. So far, its biomechanical responses have been detected by measuring torque about ankle joint. Torque about ankle joint is exerted by group of muscles - in case of muscle dystrophy weakened muscles are supported by less harmed ones - usually the extensors. Thus torque is exerted by stronger muscles, although the response of tibialis anterior muscle can be palpated. With measuring tool for measuring muscle belly displacement (displacement sensor) doctor's palpation can be replaced and improved - (1) with displacement sensor amplitude of muscle response to electrical stimulus can be measured very accurately and (2) response can be evidently presented by muscle belly displacement against time.

Measuring procedure (measurement of skeletal muscle belly displacement): displacement sensor is pressed to the skin above measured muscle belly radial to the muscle surface. In case of long and narrow skeletal muscles (e.g. m. radialis, m. fleksor digitorum superficialis, m. tibialis anterior, m. erector spinae, m. triceps brachii) measuring point is defined as a point on the skin above measured muscle where maximum displacement of muscle belly is detected. In case of large and flat skeletal muscles (e.g. m. gluteus maximus, m. soleus, m. latissimus dorsi, m. pektoralis major) measuring error is minimized if measuring point is selected according to size and position of nearby bones (Burger et al, 1997).

Measuring tool used for measuring muscle belly displacement, as described in this patent application, is inductive or optical displacement sensor. It incorporates stepper motor or metallic spring in order to provide the same initial pressure (sensor tip / skin) before tensiomyographic measurement is performed. This initial pressure is usually provided by 0.17 N/mm spring and reaches 150 x 10E-3 N/mm² on a tip area of 113 mm². Spring coefficient ranges from 0.1 to 0.5 N/mm. Thus the initial pressure (tip area: 113 mm²) ranges from 2 to 400 x 10E-3 N/mm². In order to calibrate sensor's sensitivity stimulation treshold, electrode positioning and sensor positioning have to be defined.

The term muscle belly displacement, as used in this patent application, is defined as the thickened middle part of skeletal muscle, which occurs when the muscle is contracted and thus shortened. Muscle belly displacement can be achieved either with voluntary contraction or contraction evoked by electrical stimulus/stimuli. Initial displacement for tensiomyographic measurement is 10 mm, usually measurements are performed at larger displacement. Used diameters sensor tip: 5, 10, 12, 15 and 35mm - mainly sensor tip with 12 mm in diameter is used.

It has been mentioned before that tensiomyographic measuring method enables monitoring changes of muscle belly displacement in voluntary as well as electrically evoked contraction - electrically evoked contraction features much higher level of repeatability. Electrical stimulation consists of single pulse stimulation or tetanic stimulation. The former consists of single DC electrical stimuli with their width ranging from 0.5 to 2 ms (mainly 1 ms impulses are applied) and their amplitude ranging from 10 to 50 V (voltage source) or from 5 to 25 mA (current source) above threshold, where the term threshold is defined as stimulation voltage that induces muscle belly displacement big enough to measure. The latter - tetanic stimulation - is a set (train) of 0.1 ms DC electrical stimuli. Pulse amplitude is adjusted to measured subjects, pulse frequency ranges from 7 to 40 Hz - depending on the process observed: fatigue process, unfused or fused tetanus. Tetanic stimulation induces a series of contractions. Increased stimulating frequency means shorter period of time between two stimuli and at some point muscle cannot relax completely - before it is completely relaxed next stimulus is generated and next contraction starts. Because the next contraction started in non-relaxed muscle, muscle belly displacement increases. This process (unfused tetanus) is repeated until stimulation frequency reaches certain value - tetanic frequency. At tetanic frequency time period between two stimuli is too short for a muscle to relax and it remains contracted throughout the stimulation - this process is called fused tetanus.

The previous studies have revealed that tetanic frequency differs with slow and fast skeletal muscles (slow muscle comprises more type I than type II muscle fibres) - compared to fast muscle, tetanic frequency with slow muscle is lower. At stimulation frequency around 8 Hz type I and type II muscle fibres can be observed separately - tetanus in type I muscle fibres is already fused while in type II muscle fibres tetanus is still unfused. Performing tetanic stimulation for a longer period of time (approx. 60 s) causes type II muscle fibres to fatigue which results in decreased muscle belly displacement (Ker 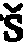 evan 1999).

Measuring technique for detection of muscle belly displacement has been evaluated with torque measuring technique (Burger et al., 1997, Kogov 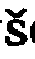 ek, 1993) and electromyographic measuring technique (EMG) (Kogov 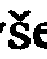 ek, 1991, Knez et al., 1999):
muscle torque (exerted by group of muscles) was compared to muscle belly displacement (of muscles in the same group) at increasing stimulation voltage. Comparison revealed that muscle belly displacement is proportional to muscle torque (exerted by the same muscle) and that maximum muscle torque is approximately proportional to maximum muscle belly displacement (as a response to single electrical stimulus);
in voluntary contraction ( (1) ramp pattern - contraction/relaxation, (2) retaining three different levels of muscle belly displacement) integrated value of EMG signal and
muscle belly displacement were compared. The study revealed that (1) muscle belly displacement is proportional to integrated EMG (the latter increased in fatigued muscle) and (2) that there is a functional correlation between muscle belly displacement and integrated EMG in static load (Knez et al., 1999).

From preliminary studies it has been concluded that TMG based parameter contraction time is correlated with velocity parameter, as defined in (Valen 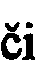 i 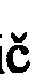 and Knez, 1997). Contraction time is defined as a period of time in which muscle belly displacement value increases from 10 to 90% of its maximum value. Within the framework of Mrs. R. Dahmane, M.Sc. doctoral thesis (University of Ljubljana, Medical Faculty) histochemical and biomechanical relationship of seven limb muscles in two groups of 15 men was examined. Statistical analysis of the data obtained by tensiomyographic and histochemical techniques shows a significant correlation between the contraction time of muscle response measured by TMG and the percentage of type I muscle fibres (correlation coefficient equals 0.93, α<0.05).

Measuring procedure and measuring device, both subjects of invention, enable measurements of biomechanical and dynamic properties of all surface skeletal muscles, among which is also latissimus dorsi muscle. This muscle is used in cardiology as a heart-assistant muscle. In the phase of preparation for transplantation, monitoring biomechanical properties of latissimus dorsi muscle is very important (Gustafson, 1997).
For many years the TMG method is successfully applied in clinical practice - with patients with neuromuscular diseases (Knez et al., 1999) and with patients after above-knee amputation (Burger, 1995, Burger et al., 1997).
In the last few years TMG method is also used by Slovene professional sport trainers in order to improve their training techniques (Valen 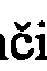 i 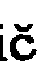 et al., 1998, Valen 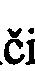 i 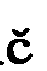 & Godina, 1998, Djordjević et al., 1998, Knez et al., 1999). Mainly the impact of fatigue on biomechanical and dynamic properties of skeletal muscles is studied (Praprotnik et al., 1999).

In the following paragraph the subject of invention is described in greater detail. This description is based on three figures, which are part of this patent application as well.

In Figure 1 the device-scheme is presented. Measuring device consists of (1) pulse generator, two self-adhesive electrodes for bipolar electrical stimulation (2, 3), displacement sensor (4) moving part of the sensor (5) sensor tip (6), electronic transducer (7), microprocessor (8), a pair of conductors (9), skeletal muscle belly (10).

In Figure 2 TMG-based parameters (listed in Table 1) are presented.

**Table 1.**

| Parameter | Description | Unit |
|---|---|---|
| T_{d} | Delay time | ms |
| Tᵢ | Beginning time | ms |
| T_{c} | Contraction time | ms |
| Tₛ | Sustain time | ms |
| Tᵣ | Relaxation time | ms |
| T_{b} | Time in which dₘₐₓ is reached | ms |
| AOI | Time ratio | % |
| AVI | Amplitude ratio | % |

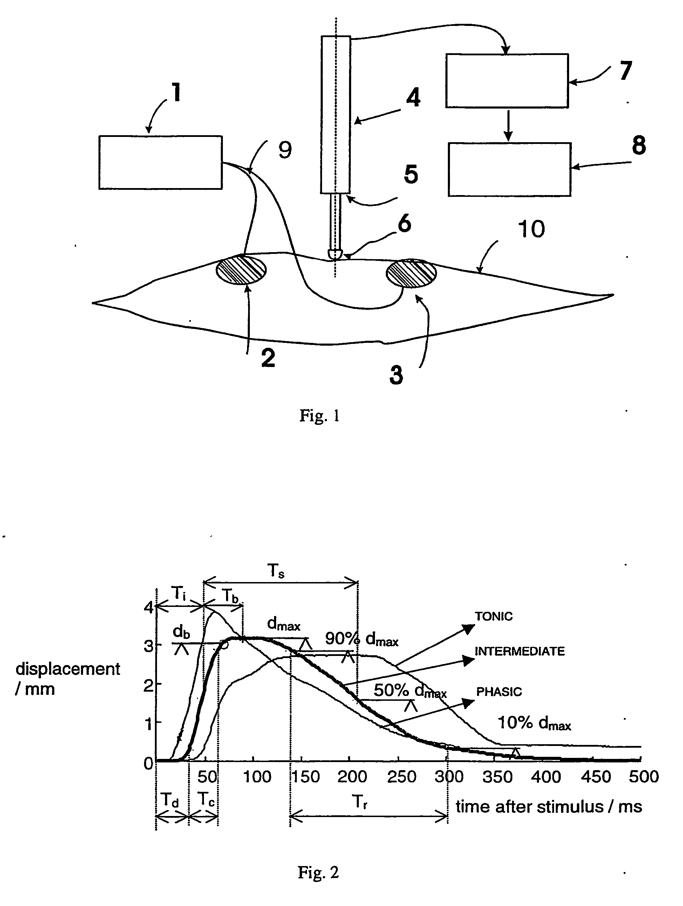

On the figure 2 the curves for tonic, intermediate and phasic muscles are shown. Tonic muscle is muscle with mainly slow muscle fibres. Intermediate muscle is muscle with comparative number of fast and slow muscle fibres. Phasic muscle is muscle with mainly fast muscle fibres.

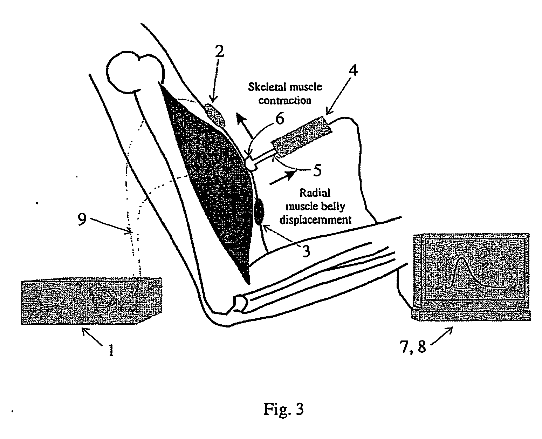

In Figure 3 position of measuring subject during the measurement is shown. On the figure is evident (1) pulse generator, two self-adhesive electrodes for bipolar electrical stimulation (2, 3), displacement sensor (4) moving part of the sensor (5) sensor tip (6), electronic transducer (7), microprocessor (8), a pair of conductors (9).

Tensiomyographic measuring procedure starts with determination of measuring point on muscle belly (Fig.1 (10)) in order to measure its radial displacement. This is followed by electrode (Fig.1 (2) and (3)) positioning. Bipolar electrical stimulation consists of single DC pulses generated by current source, for purposes of this application denoted also pulse source or pulse generator (Fig.1 (1)), connected to electrodes by both conductors (Fig.1 (9)). Measuring data (TMG response) are collected by electronic transducer inside computer (Fig.1 (7)) and processed by computer software (Fig.1 (8)), which performs statistical analysis and calculates TMG based parameters.

In case of monopolar electrical stimulation tensiomzographic measuring device is realized with integrated electrode. This electrode is composite part of the sensor (Fig.1 (6)), made of surgical stainless steel and covered with polyurethane, which serves as isolation.

In order to perform measuring procedure (contractile properties of surface skeletal muscles detection) as described above, the measuring device (operative mode) has to comprise as follows: at least one current source for generating pulse-trains (Fig.1 (1)), at least one pair of stimulation electrodes (Fig.1 (2) and (3)), at least one displacement sensor (Fig.1 (4)) (differential transformer or optical realization), electronic transducer for data measured in time space by displacement sensor (Fig.1 (7)), computer or microprocessor (Fig.1 (8)) for calculating TMG based parameters.

Measuring device - the subject of invention - currently in use comprises a current source (Fig.1 (1)), which generates single DC pulse-trains of 0.01 - 50 Hz. Single DC pulses are of 0.1- 100 ms duration and 1 - 110 mA.

Moving component (Fig.1 (5)) of displacement sensor currently in use (Fig.1 (4)) detects muscle belly displacements between 5 and 70 mm. Tip (Fig.1 (6)) diameter ranges from 2 to 40 mm. Displacement sensor incorporates a spring (spring coefficient 0.1 - 0.5 N/mm) for providing initial pressure (usually between 0.1 N and 10 N) on the skin above the measured skeletal muscle. In order to achieve maximum repeatability the initial displacement of the moving component should be set to 0.1- 20 mm.

Electrode positioning in TMG measuring procedure: in bipolar electrical stimulation the anode is usually placed distally and the cathode proximally, 20 - 50 mm from measuring point; in monopolar stimulation one electrode is placed on the muscle belly and the other on the limb distally from muscle belly

TMG based parameters (Fig.2 and Table 1) in twitch contractions are as follows: maximum displacement value (dₘₐₓ) - maximum value of skeletal muscle displacement (TMG response), delay time (*T_{d}*) *-* a period of time in which the TMG response increases from 0 to 10% dₘₐₓ, contraction time (*T_{c}*) - a period of time in which the TMG response increases from 10 to 90% dₘₐₓ, sustain time (*Tₛ*) - a period of time in which TMG response is greater than 50% dₘₐₓ, relaxation time (*Tᵣ*) - a period of time in which the TMG response decreases from 90 to 10% dₘₐₓ, *T_{b}* - a period of time in which dₘₐₓ is reached, AOI - time ratio and AVI - amplitude ratio.
The above-mentioned parameters are shown in Fig. 2 and are the subject of this patent application as well.

TMG measuring device can be constructed using standard component parts, such as (1) Dc-Dc magnetic transducer produced by Trans-Tek, Inc., model #0353-000, where its original tip has to be replaced with the one of 8 mm in diameter. In order to assure optimal measuring conditions the initial displacement should set to 5 -15 mm;
(2) for electronic transducer inventors suggest to use analogue-digital transducer as applied in regulation technique;
(3) for data processing the usual computer processor can be used.

Calibration of TMG measuring device is based on initial pressure of approximately 1.07 N/cm². If the device consisted of other components than suggested by inventors and if the initial pressure of approximately 1.07 N/cm² was provided, the main element of the subject of invention would not change and measuring results would still be reliable.

## Claims

1. Method for selective and non-invasive detection of skeletal muscles contraction process for improving training techniques in sports **characterized in that** it comprises steps of
- positioning of a displacement sensor on a skin above a measured muscle,
- stimulation of said measured skeletal muscle to evoke contraction of said measured skeletal muscle, said stimulation comprised of an external stimulus by at least one electrical impulse, and
- measurement of said muscle belly radial displacement, said muscle belly displacement a result of muscle contraction,
whereas muscle work is essentially proportional to the square of said muscle belly displacement as a result of said skeletal muscle contraction.

2. Method according to claim 1 **characterized by** stimulation frequency approximately equal to tetanic frequency whereby the average velocity of muscle response is defined.

3. Method according to claims 1 **characterized by** defining of muscle velocity by measurement or plurality thereof of contraction time, said velocity related to muscle fibres types.

4. Method according to claim 1 **characterized by** stimulation provided by wilful stimulation of said measured muscle, said stimulation exercised by subject of measurement.

5. Method according to claim 1 **characterized by** measuring procedure which is applicable particularly for measurements under isometric conditions.

6. Method according to claim 1 **characterized in that** in a combination with mechanical braces and/or other measuring devices within commercial reach said method is applicable for measurements under isotonic and/or isokinetic conditions.

7. Method according to claim 1 **characterized by** positioning of the measuring device for detection of radial skeletal muscle belly displacement is performed with respect to measured muscles shape and size: in case of long and narrow skeletal muscles (*e*.*g*. m. *radialis, m. fleksor digitorum superficialis, m. tibialis anterior, m. erector spinae, m. triceps brachii*) measuring point is the point on the skin above measured muscle where maximum belly enlargement is expected, and in case of large flat muscles (e.g. *m*. *gluteus maximus, m. soleus, m. latissimus dorsi, m. pektoralis major*) measuring point is selected according to size and position of nearby bones.

8. A computer program product directly loadable into an internal memory of a digital computer, comprising software code portions for performing the steps of any preceding claims when said product is run on a computer.

9. Device for selective and non-invasive measurements of muscle force and/or muscle torque **characterized in that** it comprises at least one current source for generating pulse-trains, at least one pair of stimulation electrodes, at least one displacement sensor, a sampling unit for sampling a signal from said displacement sensor, and processing means for processing said signals.

10. Device for selective and non-invasive detection of a skeletal muscle contraction - process and/or for indirect measurements of said muscle force and/or said muscle torque **characterized by** measurement means for detection of said skeletal muscle belly displacement, stimulation means for electrical stimulation of said skeletal muscle contraction and regulation means for providing essentially constant initial pressure on a skin above said measured muscle belly before each measurement.

## Patentansprüche

1. Selektive und nicht-invasive Methode zur Untersuchung von Skelettmuskelkontraktionsprozesses, **gekennzeichnet** mit folgenden Verfahrenschritten:
- Aufsetzung von Verformungssensor an die Haut oberhalb der gemessenen Muskel
- Stimulierung von benannten gemessenen Muskel um Kontraktion von benannten gemessenen Muskel hervorzurufen, die benannte Stimulierierung umfasst mindestens ein elektrisches Impuls, und
- Messung der Verformung des Muskelbauches, die benannte Verformung des Muskelbauches als Resultat von Muskelkontraktion,
wobei die Muskelaktivität im wesentlichen proportional dem Quadrat des Verformungs des Muskelbauches ist, als Resultat von benannten Muskelkontraktion.

2. Methode im Anspruch 1 kennzeichnet eine Stimulationsfrequenz aus, die etwa der tetanischer Frequenz entspricht und die die durchschnittliche Reaktionsgeschwindigkeit (Verzögerungszeit) des Muskels definiert.

3. Methode im Anspruch 1 kennzeichnet eine Kontraktionsgeschwindigkeit erhoben mit Messungen von Kontraktionszeiten, wobei die benannte Geschwindigkeit eng mit der Muskelzusammensetung koreliert ist.

4. Methode im Anspruch 1 kennzeichnet bewusste Stimulation von benannten gemessenen Muskel, wo die benannte Stimulation von einem Messer ausgeführt wird.

5. Methode im Anspruch 1 kennzeichnet ein Messverfahren, der besonders für Messungen unter isometrischen Bedingungen anwendbar ist.

6. Methode im Anspruch 1 ist kennzeichnet damit es in der Kombination mit mechanischen Instrumenten und anderen herkömmlichen Messgeräten, die benannte Methode besonders für Messungen unter isotonischen und/oder isokinetischen Bedingungen anwendbar ist.

7. Methode im Anspruch 1 kennzeichnet das Positionieren der Messsonde zur Messung der Verformung des Muskelbauches, wobei die Form und Grösse der gemessenen Muskalatur berücksichtigt werden: im Fall von langen und schmalen Muskeln (z.B. *m. radialis, m. fleksor digitorum superficialis, m. tibialis anterior, m. erector spinae, m. triceps brachii*) wird die Messstelle an der Haut oberhalb von gemessenen Muskel, wo die maximale Verformung des Muskelbauches erwartet wird; im Fall von grossen, flachen Muskeln (e.g. *m. gluteus maximus, m. soleus, m. latissimus dorsi, m. pektoralis major*) wird die Messstelle in Hinsicht auf Grösse und Lage von nahliegenden Knochen gewählt.

8. Die gewonnenen Daten werden digitalisiert direct in den Computer geschickt, der die Schritte der vorerwähnten Ansprüchen mit Hilfe einer speciellen Software prozessiert.

9. Das Gerät für selektive und nicht-invasive Messungen des Volumens und/oder des Tonus der benannten Muskel ist damit kennzeichnet, dass es mindestens ein Impulsgeber, ein Paar von Elektroden, ein Verformungssensor, eine Messonde, die den Signal aus dem Verformungssensor aufzeichnet, und ein Computer, der den benannten Signal auswertet, einschliesst.

10. Das Gerät für selektive und nicht-invasive Untersuchung von Skelettmuskelkontraktionsprozesses und für indirekte Messungen des Volumens und/oder des Tonus der benannten Muskel kennzeichnet ein Verformungssensor, der die selective Verformung der benannten Muskel mist, eine Stimulationseinheit zur elektricher Stimulierung der benannten Skelettmuskelkontraktion und ein Drucksensor, der einen konstanten Anfangsdruck auf die Haut oberhalb von gemessenen Muskel ausübt.

## Revendications

1. La méthode pour la détection sélective et non envahissante du processus de la contraction des muscles squelettiques pour amélioration des techniques d'entraînement sportif **caractérisée par** les étapes suivantes :
• Le placement du détecteur de déplacement sur la peau au-dessus du muscle mesuré,
• La stimulation du muscle squelettique mesuré pour provoquer sa contraction en comprenant le stimulus externe par au moins un impulsion électrique ; et
• La mesure du déplacement radial du ventre du muscle, le déplacement étant le résultat de la contraction du muscle, tandis que le mouvement du muscle est avant tout proportionnel au carré du déplacement du ventre du muscle en résultant de la contraction du muscle.

2. La méthode suivant l'affirmation 1, **caractérisée par** la fréquence de la stimulation, celle-ci étant environ égale à la fréquence tetanic. Ainsi la vitesse de la réaction du muscle est déterminée.

3. La méthode suivant l'affirmation 1, **caractérisée par** la définition de la vitesse du muscle en la mesurant ou par la pluralité de la durée de la contraction. La vitesse ayant la relation aux types des fibres musculaires.

4. La méthode suivant l'affirmation 1, **caractérisée par** la stimulation fournie par la stimulation délibérée du muscle mesuré. La stimulation est exercée par le sujet de la mesure.

5. La méthode suivant l'affirmation 1, **caractérisée par** la procédure de la mesure, particulièrement pour effectuer des mesures dans des conditions isométriques.

6. La méthode suivant l'affirmation 1, **caractérisée par** la combinaison de l'appareil mécanique et/ou autres appareils de mesure disponibles au marché. La méthode peut être utilisée pour effectuer des mesures dans des conditions isotoniques et/ou isokinetic aussi bien.

7. La méthode suivant l'affirmation 1, **caractérisée par** le placement de l'appareil de mesure pour le déplacement radial du ventre du muscle squelettique est effectuée à l'égard de la forme et de la taille du muscle mesuré: en cas de muscles squelettiques longs et allongés (par exemple: m. radialis, m. fleksor digitorum superficialis, m. ribialis anterior, m. erector spinae, m. triceps brachii) le point de mesure se trouve sur la peau au-dessus du muscle mesuré où la dilatation du ventre du muscle maximum est attendu. En cas de muscles grands et plats (par exemple: m. gluteus maximus, m. soleus, m. latissimus dorsi, m. pktoralis major) le point de mesure est choisi selon la taille et la position des os qui se trouvent près du muscle.

8. Le programme informatique est chargeable directement en mémoire interne du calculateur numérique. Le programme consiste en parties de la code du logiciel, nécessaires pour mettre les étapes d'affirmations précédentes à exécution pourvu que le produit soit exécuté sur l'ordinateur.

9. L'appareil pour la mesure sélective et non envahissante de la force de muscle et/ou le serrage de muscle, **caractérisé par le fait qu'**il comprend au moins une source de courant pour la production du train d'impulsion, au moins une paire d'électrodes pour la stimulation, au moins un détecteur de déplacement, un échantillon pour le prélèvement du signal du détecteur de déplacement et les moyens de traitement pour les signaux mentionnés.

10. L'appareil pour la détection sélective et non envahissante du processus de la contraction des muscles squelettiques et/ou pour la mesure indirecte de la force de muscle et/ou le serrage de muscle, **caractérisé par** les moyens de mesure pour la détection du déplacement du ventre du muscle squelettique, par les moyens de stimulation électrique pour la contraction de muscles squelettiques et enfin par les moyens du réglage pour fournir la pression constante et essentielle sur la peau au-dessus du ventre du muscle avant chaque mesure.
